Europäisches Patentamt

European Patent Office

Office européen des brevets

(11) Veröffentlichungsnummer: **0 278 107**
**B1**

(12) **EUROPÄISCHE PATENTSCHRIFT**

(45) Veröffentlichungstag der Patentschrift:
**30.05.90**

(51) Int. Cl.⁵: **C07C 59/105**, C07C 51/285

(21) Anmeldenummer: **87119093.0**

(22) Anmeldetag: **23.12.87**

(54) Verfahren zur Herstellung von Aldonsäuren oder deren Salze.

(30) Priorität: **11.02.87  DE 3704166**

(43) Veröffentlichungstag der Anmeldung:
**17.08.88 Patentblatt 88/33**

(45) Bekanntmachung des Hinweises auf die Patenterteilung:
**30.05.90 Patentblatt 90/22**

(84) Benannte Vertragsstaaten:
**AT BE CH DE ES FR GB GR IT LI LU NL SE**

(56) Entgegenhaltungen:
**DE-B- 1 244 151**
**DE-C- 461 370**
**US-A- 2 152 248**

(73) Patentinhaber: **DEUTSCHE SOLVAY-WERKE GMBH,
Langhansstrasse 6, D-5650 Solingen 11(DE)**

(72) Erfinder: **Beisecker, Dieter, Dr. Dipl.-Chem.,
Ludwigstrasse 3, D-4134 Rheinberg(DE)**

(74) Vertreter: **Seiler, Siegfried, c/o DEUTSCHE
SOLVAY-WERKE GmbH
Langhansstrasse 6 Postfach 11 02 70,
D-5650 Solingen 11(DE)**

**Beschreibung**

Die vorliegende Erfindung betrifft ein Verfahren zur Herstellung von Aldonsäuren oder deren Salze durch Oxidation von mindestens einer Aldose oder einem Derivat einer Aldose.

In den Literaturstellen Ind. J.Technology 5 (5), 152 - 4 (1967) und Ind. J. Technology 6 (5) 146 - 9 (1968) wird die elektrochemische Oxidation von Lactose als Aldose zu Calciumlactobionat als Salz einer Aldonsäure mit Brom in Gegenwart von Natriumhydrogencarbonat in einer elektrolytischen Zelle mit stationärer oder rotierender Elektrode beschrieben.

Für eine derartige elektrochemische Oxidation stehen die erforderlichen Anlagen jedoch nicht überall zur Verfügung und die Durchführung elektroorganischer Synthesen erfordert aufwendige Investitionen.

Aus der DE-OS 20 38 230 ist ein Verfahren zur Gewinnung von Lactulose und Lactobionsäure aus Lactose bekannt, in dem Lactose im alkalischen Medium zunächst zu Lactulose isomerisiert und anschließend die nicht isomerisierte, verbleibende Lactose mittels Lactose-Dehydrogenase enzymatisch zu Lactobionsäure oxidiert und letztere von der Lactulose mittels basischer Ionenaustauscher oder durch Fällen als basisches Calciumsalz abgetrennt wird. Bei dieser enzymatischen Oxidation ist jedoch die Kultur und Gewinnung bestimmter Enzymstämme erforderlich.

Gemäß der DE-PS 461 370 erfolgt die Oxidation der Aldosen in alkalischer Lösung mit Hypochlorit in Gegenwart geringer Mengen geeigneter Brom- oder Jodverbindungen.

In der DE-PS 473 261 wird ein Verfahren zur Herstellung von Monocarbonsäuren aus Aldosen wie Glucose, Galactose, Mannose, Lactose und Maltose beschrieben. Hierbei werden die Aldosen in alkalicarbonatalkalischer Lösung in Gegenwart geringer Mengen von Brom- oder Jodverbindungen mit Chlor oxidiert. Eine Nacharbeitung der darin angegebenen Arbeitsvorschrift wurde mit Lactose als Aldose wie folgt durchgeführt:

Lactose wurde als 50 %ige Suspension vorgelegt, mit Natriumbromid versetzt und Soda zugegeben, bis sich der pH-Wert 9,6 einstellte. Während der bei 50 °C durchgeführten Oxidation fällt der pH-Wert ab und wird durch portionsweise Zugabe von Soda auf pH 8 - 8,5 einreguliert. Nach Abbruch der Reaktion wird der pH-Wert durch überschüssiges Chlor auf pH 7 eingestellt. Theoretisch werden für den Umsatz von einem Mol Lactose zu einem Mol Na-Lactobionat 1,5 Mol $Na_2CO_3$ und ein Mol Chlor verbraucht.

Es zeigt sich, daß bei einem Verbrauch von 1,5 bzw. 2,16 Mol $Na_2CO_3$ und mindestens der durch Soda äquivalenten Menge Chlor pro Mol Lactose 84,6 bzw. 96,2 % der Lactose oxidiert wurden (Bestimmung der Rest-Lactose nach der Chloramin T-Methode). Wegen des hohen Salzanteils der Reaktionslösung wurde diese mit Wasser verdünnt (bis sie 20 %ig an ursprünglich eingesetzter Lactose war) und dann das Na-Lactobionat stufenweise mit Ethanol aus 50 bis 60 %iger alkoholischer Lösung gefällt. Die so erhaltene Ausbeute betrug 49,4 % bzw. 66 % der Theorie, bezogen auf die zu Beginn der Oxidation eingesetzte Lactose. Der sich in alkalischer Lösung umsetzende Anteil des Chlors wird als Natriumchlorid, Natriumhypochlorit oder Natriumchlorat gebunden. Aus den vorstehend genannten Sodamengen und dem zur Reaktionslösung zugegebenen Natriumbromid errechnet sich pro Tonne eingesetztes Lactose-Monohydrat eine Salzbelastung des Reaktionsgemisches von rund 500 bis 730 kg.

Dies erschwert nicht nur die Isolierung von reinem Natriumlactobionat, sondern führt auch zu einer erheblichen Abwasserbelastung.

Die Umwandlung des Natriumlactobionats in das für manche Anwendungszwecke interessante Calciumlactobionat muß über einen Kationenaustauscher erfolgen.

Im Falle einer Oxidation der Lactose mit Chlor in einer Lösung, die $Ca^{2+}$-Ionen enthält, bildet sich zwangsläufig ein Doppelsalz des Calciumlactobionats mit Calciumchlorid, wodurch eine unmittelbare Isolierung des reinen Calciumlactobionats erschwert wird. Dieses Doppelsalz muß daher durch Fällen eines basischen Calciumlactobionats zerlegt werden, aus dem in zweiter Stufe durch Umsetzung mit $CO_2$ Calciumlactobionat neben $CaCO_3$ erhalten wird.

Der vorliegenden Erfindung liegt daher die Aufgabe zugrunde, ein einfaches, wirtschaftliches Verfahren zur Herstellung von Aldonsäuren oder deren Salze zu finden, bei dem eine Belastung des Abwassers und des Endproduktes mit Fremdsalzen weitgehend vermieden und eine erleichterte Isolierung der Aldonsäuren aus deren Salzen über Kationenaustauscher ermöglicht wird.

Zur Lösung dieser Aufgabe ist das eingangs beschriebene Verfahren der Herstellung von Aldonsäuren oder deren Salzen durch Oxidation von mindestens einer Aldose dadurch gekennzeichnet, daß mindestens eine Aldose durch jodkatalysierte und/oder durch mindestens einer jodhaltigen Verbindung katalysierte Oxidation mit mindestens einem Peroxid oder mindestens einer peroxidgruppenhaltigen Verbindung, vorzugsweise Wasserstoffperoxid, zu der bzw. den Aldonsäure(n) bzw. deren Salz(en) bei einem pH-Wert unter 7 bzw. in einem pH-Bereich unterhalb 7 oxidiert wird.

Die Oxidation der Aldosen mit Wasserstoffperoxid allein führt im sauren, neutralen oder alkalischen pH-Bereich zu unbefriedigenden Ergebnissen. Der hierbei auftretende, stechende Geruch (vermutlich Ameisensäure) deutet nämlich auf eine Bayer-Villiger-Umlagerung der Aldosen hin. Dagegen wird durch die erfindungsgemäße Oxidation der Aldosen mit Wasserstoffperoxid bei einem pH-Wert unter 7 bzw. einem pH-Bereich unterhalb 7 die Bildung dieser Umlagerungsprodukte vermieden.

Innerhalb des beanspruchten Verfahrens werden zur Oxidation pro Mol Aldose 1,1 bis 10 Mol, vorzugsweise 1,5 bis 6 Mol Peroxid oder peroxidgruppenhaltige Verbindung, vorzugsweise Wasserstoffperoxid, eingesetzt.

Nach einer Ausführungsform erfolgt die durch Jod und/oder mindestens einer jodhaltigen Verbindung katalysierte Oxidation mit mindestens einem Peroxid oder einer peroxidgruppenhaltigen Verbindung in Abhängigkeit von dem jeweiligen Druck und/oder der jeweiligen Verfahrenstemperatur innerhalb eines pH-Bereiches von 3 bis 6,9, vorzugsweise 4 bis 5,5.

Gemäß einer weiteren Ausführungsform wird die Oxidation der Aldosen in Abhängigkeit von dem jeweiligen Druck bei Temperaturen von 313 bis 443 K, vorzugsweise 333 bis 363 K, durchgeführt.

Nach einer vorteilhaften Ausführungsform besteht der Katalysator aus Jod und mindestens einer, die Löslichkeit von Jod herbeiführenden Verbindung oder einer jodhaltigen, innerhalb des Verfahrens jodbildenden Verbindung. Die Verbindung besteht vorzugsweise aus Jodwasserstoffsäure und/oder deren wasserlöslichen oder im Reaktionsmedium löslichen Salze.

Gemäß einer zweckmäßigen Ausführungsform wird das Jod und/oder die jodhaltige Verbindung, z. B. Jodwasserstoff, in Gewichtsmengen unter 1/50, vorzugsweise unter 1/80, des Gesamtgewichts der Aldose(n) eingesetzt.

Die erfindungsgemäße Oxidation der Aldose(n) läßt sich auch durch einen Kreisprozeß beschreiben gemäß nachfolgendem Schema:

$$
\begin{array}{ccc}
 & + & \\
\mathrm{J_2} \longrightarrow\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\! & & \text{Aldose} \\
\uparrow & & \downarrow \\
\mathrm{H_2O_2} \longleftarrow\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\!\! & & \text{Aldonsäure} + 2\mathrm{HJ}, \\
 & + & \\
\end{array}
$$

aufgrund dessen sich das während der Reaktion zugesetzte Wasserstoffperoxid mit dem im Reaktionsgemisch gebildeten oder mit dem zugesetzten Jodwasserstoff umsetzt.

Gemäß einer besonders vorteilhaften Ausführungsform des Verfahrens wird bzw. werden die nach der Oxidation der Aldose(n) verbleibende(n) Jodverbindung(en) durch zusätzlich zugeführte Oxidationsmittel, vorzugsweise Wasserstoffperoxid, zu Jod oxidiert und zusammen mit evtl. nicht umgesetztem Jod zurückgewonnen, vorzugsweise zur durch Jod katalysierten Oxidation in das Verfahren zurückgeführt.

Zur Oxidation der Aldose wird nach einer vorteilhaften Ausführungsform ein Jod/Jodwasserstoff-Gemisch oder nach einer weiteren Ausführungsform Jodwasserstoff allein verwendet. Nach vollständigem Umsatz der Aldose wird der Jodwasserstoff mit Wasserstoffperoxid zu Jod oxidiert, das Jod restlos abgetrieben, in einer Vorlage aufgefangen und so seine Rückführung in den Reaktor ermöglicht.

Innerhalb des erfindungsgemäßen Verfahrens wurden als Beispiele für Aldosen oder deren Derivate Glucose, Galactose und Lactose zu den entsprechenden Aldonsäuren bzw. deren Salzen oxidiert. Das erfindungsgemäße Verfahren hat jedoch eine über die spezielle Gewinnung von Lactobionsäure, Galactonsäure und Gluconsäure bzw. deren Salzen hinausgehende Bedeutung, da es sich auch auf andere Aldosen oder Derivaten der Aldosen anwenden läßt.

Im Gegensatz zur bisher üblichen Oxidation mit Chlor werden durch das erfindungsgemäße Verfahren nur geringe Mengen Fremdsalze in das Abwasser oder in das Endprodukt eingeschleppt.

Beispiel 1: Herstellung von Calciumlactobionat

Vor dem Beschicken wird das Reaktionsgefäß mit Stickstoff durchspült und das Inertgas während der gesamten Reaktionszeit durch den Ansatz geleitet.

727 g (2 mol) 99 %iges Lactose-Monohydrat als Aldose werden bei 353 K in 890 g Wasser gelöst, erforderlichenfalls mit Aktivkohle entfärbt und eingedampft, bis eine 60%ige Lösung vorliegt.

Als Katalysator werden 6,6 ml 57 %ige Jodwasserstoffsäure (0,025 mol HJ pro mol Lactose) verwendet. Statt Jodwasserstoffsäure kann auch ein Gemisch aus Jod und Jodwasserstoffsäure verwendet werden.

Zur vorgelegten warmen Lactose-Lösung tropft man nebeneinander Jodwasserstoffe und eine Calciumhydroxidlösung so zu, daß sich ein pH-Wert von 5 einstellt.

Die nicht benötigte Jodwasserstoffsäure kann im Verlauf der Reaktion zugeben werden, soweit dies als notwendig erscheint.

Zur jodhaltigen Lactose-Lösung läßt man anschließend eine 30 %ige Wasserstoffperoxidlösung zutropfen und regelt die Zugabegeschwindigkeit in der Weise, daß einerseits genug Jod freigesetzt wird, andererseits auch nicht zu viel Joddampf entsteht.

Entweichendes Jod wird in Wasser und kalkmilchhaltigen Vorlagen aufgefangen und kann so wieder verwendet werden. Den während der Lactose-Oxidation abfallenden pH-Wert hält man durch geeignete Kalkmilchdosierung im Bereich von 4,5 - 5.

Zur Unterdrückung einer Hydrolyse und zur Beschleunigung des Reaktionsablaufs führt man die Oxidation im Konzentrationsbereich von 45 bis 60 Gew.-% durch (bezogen auf das eingesetzte Lactose-Monohydrat). Dies kann man durch Zwischeneindampfung in Vakuum bewerkstelligen.

Vor jeder Eindampfung stoppt man die Wasserstoffperoxidzuführung, damit das im Gemisch vorhandene Jod durch die Aldose zu Jodid reduziert wird.

Die Lactose-Oxidation ist in den ersten drei Stunden deutlich exotherm. In dieser Zeit werden 80 % der Disaccharose oxidiert. Jedoch fällt die Reaktionsgeschwindigkeit asymptotisch ab. Der noch vorhandene Lactose-Anteil kann entweder jodometrisch bestimmt werden, indem man den Proben Kaliumjodid zusetzt, dieses durch Chloramin zu Jod oxidiert und nach Oxidation der Restlactose Jod zurücktitriert oder enzymatisch bestimmt werden, indem nach enzymatischer Spaltung der Lactose und enzymatischer Oxidation der so freigesetzten Galactose, die Lactose in Proben durch Extinktionsmessung des Coenzyms NaDH im langwelligen UV-Bereich bestimmt wird.

Eine etwaige Hydrolyse der Lactose läßt sich durch enzymatische Bestimmung der gebildeten Galactose feststellen.

Normalerweise tritt jedoch eine Hydrolyse von Lactose bzw. Calciumlactobionat in den ersten 10 Stunden der Oxidation nicht auf, wenn wie vorstehend beschrieben verfahren wird.

Nach ca. 10 bis 12 stündiger Oxidation, neben gelegentlicher Zwischeneindampfung, unterbricht man die Reaktion, wenn nur noch wenig Restlactose in der Lösung vorhanden ist. Das im Reaktionsgemisch vorhandene Jodid wird durch zutropfendes Wasserstoffperoxid zu Jod oxidiert, dieses im Vakuum verdampft und in Vorlagen aufgefangen. Die Vertreibung des Jods wird durch gelegentliche Wasserstoffperoxidzugabe bzw. bei nur geringen Jodmengen nach Verkochen des Wasserstoffperoxids mit Kaliumjodidstärkepapier überprüft.

Anschließend wird das Reaktionsgemisch mit Aktivkohle behandelt, im Vakuum eingedampft und der zunächst entstehende sirupöse Rückstand im Hochvakuum so scharf getrocknet, daß er pulverisierbar wird. Der pulverisierte Eindampfrückstand enthält neben Calciumlactobionat etwa 5 % Wasser, etwa 1,8 % Lactose, etwa 0,5 % Galactose und etwa 0,5 % Glucose neben etwa 0,01 % Jod. Ausbeute: 70 % der Theorie, berechnet als wasserfreies Calciumlactobionat nach Abzug der vorgenannten Nebenbestandteile.

Schickt man eine Lösung dieses Calciumlactobionats durch einen mit $H^+$-Ionen beladenen Kationenaustauscher, so erhält man als Aldonsäure die entsprechende Lactobionsäure.

Zwischeneindampfungen und Jodaustreibung können im Vakuum im Dünnschichtverdampfer durchgeführt werden, wobei Eindampfung und Jodaustreibung in einem einzigen Arbeitsgang durchgeführt werden können. Falls die Endkonzentration schon vor der Jodentfernung erreicht ist, läßt man das Konzentrat im Dünnschichtverdampfer umlaufen und hält den Wasseranteil durch entsprechende Wasserzuführung konstant.

Die anschließende Endeindampfung geschieht vorteihaft im Sprühtrockner. Das Calciumlactobionat fällt hierbei pulverförmig an.

Verwendet man zur Neutralisation der bei der eingangs beschriebenen Oxidationsreaktion entstehenden Lactobionsäure eine Natriumhydroxidlösung anstelle der Calciumhydroxidlösung, so erhält man das Natriumlactobionat.

Beispiel 2: Herstellung von Calciumgluconat

595 g D(+)-Glucose-Monohydrat als Aldose werden bei 353 K in 307 g Wasser gelöst. Die Oxidation, pH-Wert-Regulierung und Aufarbeitung des Reaktionsgemisches entsprechen den Bedingungen des Beispiels 1. Der pH-Wert des Reaktionsgemisches wird bei ca. 4,5 gehalten. Nach sechsstündiger Oxidationszeit und einer Zwischeneindampfung beginnt die Ausfällung von Calciumgluconat.

Nach nochmals 5,5 Stunden Oxidation und einer Zwischeneindampfung erhält man eine Fällung von 532 g Reaktionsprodukt, welches neben kristallisiertem und vorwiegend hydratisiertem Calciumgluconat etwa 7,5 % Glucose und etwa 0,21 % Jod enthält. Wasseranteil: 3,29 %. Nach Abzug der vorgenannten Nebenbestandteile beträgt die Ausbeute an Calciumgluconat 73 % der Theorie, berechnet als wasserfreies Calciumgluconat.

Die Ausbeute läßt sich durch Aufarbeiten des Filtrats noch erhöhen.

Beispiel 3: Herstellung von Calciumgalactonat

600 g D(+)-Galactose werden in 400 ml Wasser gelöst. Oxidation, pH-Wert-Regulierung und Aufarbeitung des Reaktionsgemisches entsprechen dem Beispiel 1.

4

Das Reaktionsgemisch wird durch diskontinuierlichen Zusatz einer Calciumhydroxidaufschlämmung im pH-Bereich 4,5 bis 5,5 gehalten.

Gegen Ende der Oxidationszeit läßt man den mit Ca(OH$_2$) regulierten pH-Bereich bis auf pH 6,8 ansteigen.

Nach 45,5 h Oxidationszeit und einer Zwischeneindampfung auf 60 % (bezogen auf die Ausgangsmenge Galactose) kristallisiert das Reaktionsprodukt schon bei beginnender Eindampfung aus. Nach dem Eindampfen der Mutterlauge findet sich nur noch wenig Rückstand.

Nach scharfem Absaugen des Gesamtprodukts und Vakuumtrocknung erhält man 763 g eines farblosen Produktes. Neben Caciumgalactonat enthält es noch etwa 0,85 % Galactose, weniger als 0,15 % Jod und 24 % Wasser.

Beim Trocknen dieses Produktes bei 45 °C und 1 bis 2 mbar reduziert sich der Wassergehalt auf 17,04 %. Aufgrund dieses Wasseranteils, des IR-Spektrums und des Ergebnisses der Elementaranalyse handelt es sich bei diesem Produkt um das Pentahydrat des Calcium galactonats.

Nach rechnerischem Abzug des Jod- und Galaktoseanteils beträgt die Ausbeute an Calciumgalatonat-Pentahydrat 87 % der Theorie.

## Patentansprüche

1. Verfahren zur Herstellung von Aldonsäuren oder deren Salze durch Oxidation von mindestens einer Aldose, dadurch gekennzeichnet, daß mindestens eine Aldose durch Jod und/oder mindestens einer jodhaltigen Verbindung katalysierten Oxidation mit mindestens einem Peroxid oder mindestens einer peroxidgruppenhaltigen Verbindung, vorzugsweise Wasserstoffperoxid, zu der bzw. den Aldonsäure(n) bzw. deren Salz(en) bei einem pH-Wert unter 7 bzw. in einem pH-Bereich unterhalb 7 oxidiert wird.

2. Verfahren nach Anspruch 1, dadurch gekennzeichnet, daß die durch Jod und/oder mindestens einer jodhaltigen Verbindung katalysierte Oxidation mit mindestens einem Peroxid oder mindestens einer peroxidgruppenhaltigen Verbindung in Abhängigkeit von dem jeweiligen Druck und/oder der jeweiligen Verfahrenstemperatur innerhalb eines pH-Bereiches von

3 bis 6,9, vorzugsweise

4 bis 5,5,

erfolgt.

3. Verfahren nach Ansprüchen 1 und 2, dadurch gekenneichnet, daß innerhalb des Verfahrens pro Mol Aldose 1,1 bis 10 Mol, vorzugsweise 1,5 bis 6 Mol,

Peroxid oder peroxidgruppenhaltige Verbindung, vorzugsweise Wasserstoffperoxid, eingesetzt werden.

4. Verfahren nach einem oder mehreren der Ansprüche 1 bis 3, dadurch gekennzeichnet, daß der Katalysator aus Jod und mindestens einer, die Löslichkeit von Jod herbeiführenden Verbindung oder einer jodhaltigen, innerhalb des Verfahrens jodbildenden Verbindung besteht.

5. Verfahren nach Anspruch 4, dadurch gekennzeichnet, daß die die Löslichkeit von Jod herbeiführende Verbindung aus Jodwasserstoffsäure und/oder deren wasserlöslichen oder im Reaktionsmedium löslichen Salzen besteht oder diese enthält.

6. Verfahren nach einem oder mehreren der Ansprüche 1 bis 5, dadurch gekennzeichnet, daß die Oxidation in Abhängigkeit von dem jeweiligen Druck bei Temperaturen von

313 bis 443 K, vorzugsweise

333 bis 363 K,

durchgeführt wird.

7. Verfahren nach einem oder mehreren der Ansprüche 1 bis 6, dadurch gekennzeichnet, daß das Jod und/oder die jodhaltige Verbindung in Gewichtsmengen unter 1/50, vorzugsweise unter 1/80, des Gesamtgewichtes der Aldose(n) eingesetzt wird.

8. Verfahren nach einem oder mehreren der Ansprüche 1 bis 7, dadurch gekennzeichnet, daß die nach der Oxidation der Aldose(n) verbleibende(n) Jodverbindung(en) durch zusätzlich zugeführte Oxidationsmittel, vorzugsweise Wasserstoffperoxid, zu Jod oxidiert und zusammen mit gegebenenfalls nicht umgesetztem Jod zurückgewonnen werden, vorzugsweise zur durch Jod und/oder mindestens einer jodhaltigen Verbindung katalysierten Oxidation in das Verfahren zurückgeführt wird bzw. werden.

## Revendications

1. Procédé pour la préparation d'acides aldoniques ou de leurs sels par oxydation d'au moins un aldose caractérisé en ce qu'au moins un aldose subit une oxydation catalysée par l'iode et/ou par au moins un composé contenant de l'iode à l'aide d'au moins un peroxyde ou d'au moins un composé contenant des groupes peroxydes, de préférence du peroxyde d'hydrogène, pour former le ou les acides aldoniques ou son ou ses sels, à un pH ou une zone de pH inférieur à 7.

2. Procédé selon la revendication 1 caractérisé en ce que l'oxydation catalysée par l'iode et/ou au moins un composé contenant de l'iode à l'aide d'au moins un peroxyde ou un composé contenant du peroxyde, s'effectue en fonction de la pression en cause et/ou de la température opératoire en cause, dans une plage de
pH de 3 à 6, 9, de préférence
de 4 à 5, 5.

3. Procédé selon les revendications 1 et 2 caractérisé en ce que selon le procédé, par mole d'aldose, on met en œuvre
1, 1 à 10 moles, de préférence
1, 5 à 6 moles,
de peroxyde ou de composé contenant du peroxyde, de préférence du peroxyde d'hydrogène.

4. Procédé selon une ou plusieurs des revendications 1 à 3 caractérisé en ce que le catalyseur est constitué d'iode et d'au moins un composé provoquant la solubilité de l'iode ou d'un composé contenant de l'iode, formant de l'iode dans le procédé.

5. Procédé selon la revendication 4 caractérisé en ce que le composé provoquant la solubilité de l'iode est constitué par de l'acide iodhydrique et/ou de ses sels solubles dans l'eau ou dans le milieu réactionnel ou contient ceux-ci.

6. Procédé selon une ou plusieurs des revendications 1 à 5 caractérisé en ce que l'oxydation en fonction de la pression en cause s'effectue à des températures de
313 à 443 K, de préférence
333 à 363 K.

7. Procédé selon une ou plusieurs des revendications 1 à 6 caractérisé en ce que l'iode et/ou le composé contenant l'iode, est mis en œuvre en des quantités pondérales inférieures à 1/50, de préférence inférieures à 1/80 du poids total du ou des aldoses.

8. Procédé selon une ou plusieurs des revendications 1 à 7 caractérisé en ce que le ou les composés d'iode restant après l'oxydation du ou des aldoses sont oxydés en iode par de l'agent d'oxydation alimenté en supplément, de préférence du peroxyde d'hydrogène, et est récupéré avec l'iode n'ayant éventuellement pas réagi, pour être recyclé dans le procédé de préférence vers l'oxydation catalysée par l'iode.

**Claims**

1. Method for the production of aldonic acids or their salts through oxidation of at least one aldose, characterized in that at least one aldose is oxidized by means of an oxidation catalysed by iodine and/or at least one compound containing iodine, with at least one peroxide or at least one compound containing peroxide groups, preferably hydrogen peroxide, to the aldonic acid(s) or respectively salt(s) thereof at a pH value of below 7 or respectively in a pH range of below 7.

2. Method according to Claim 1, characterized in that the oxidation which is catalysed by iodine and/or at least one compound containing iodine, takes place with at least one peroxide or at least one compound containing peroxide groups, as a function of the respective pressure and/or the respective process temperature within a pH range of
3 to 6.9 preferably
4 to 5.5.

3. Method according to Claims 1 and 2, characterized in that within the method per mole aldose
1.1 to 10 mol, preferably
1.5 to 6 mol
peroxide or compound containing peroxide groups, preferably hydrogen peroxide, are used.

4. Method according to one or more of Claims 1 to 3, characterized in that the catalyst consists of iodine and at least one compound bringing about the solubility of iodine, or a compound containing iodine, which forms iodine within the method.

5. Method according to Claim 4, characterized in that the compound which brings about the solubility of iodine consists of, or contains, hydroiodic acid and/or its salts which are water-soluble or are soluble in the reaction medium.

6. Method according to one or more of Claims 1 to 5, characterized in that the oxidation is carried out as a function of the respective pressure at temperatures of
313 to 443 K, preferably
333 to 363 K.

7. Method according to one or more of Claims 1 to 6, characterized in that the iodine and/or the compound containing iodine, is used in quantities of weight below 1/50, preferably below 1/80, of the total weight of the aldose(s).

8. Method according to one or more of Claims 1 to 7, characterized in that the iodine compound(s) remaining after the oxidation of the aldose(s) is/are oxidized to iodine by additionally provided oxidation agents, preferably hydrogen peroxide, and recovered together with iodine which may not have been converted, preferably is/are returned into the method for oxidation catalysed by iodine and/or at least one compound containing iodine.